(19) Europäisches Patentamt
European Patent Office
Office européen des brevets

(11) Publication number: **0 109 767 B1**

# (12) EUROPEAN PATENT SPECIFICATION

(45) Date of publication of patent specification: **08.06.88**

(51) Int. Cl.$^4$: **G 01 N 27/30**

(21) Application number: **83306407.4**

(22) Date of filing: **21.10.83**

## (54) Modified electrode.

(30) Priority: **09.11.82 JP 196608/82**
**21.07.83 JP 133103/83**

(43) Date of publication of application:
**30.05.84 Bulletin 84/22**

(45) Publication of the grant of the patent:
**08.06.88 Bulletin 88/23**

(84) Designated Contracting States:
**DE FR GB**

(56) References cited:
**EP-A-0 056 283**
**EP-A-0 083 861**
**EP-A-0 088 592**
**DE-A-3 148 366**
**US-A-4 144 143**

**CHEMICAL ABSTRACTS, vol. 96, 1982, page 508, no. 76351t, Columbus, Ohio, US A. TORSTENSSON et al.: "Catalytic oxidation of NADH by surface-modified graphite electrodes"**

(73) Proprietor: **AJINOMOTO CO., INC.**
**5-8, Kyobashi 1-chome, Chuo-ku**
**Tokyo 104 (JP)**

(72) Inventor: **Kimura, Yasuhiro**
**No. 2-20-8, Kannon Kawasaki-ku**
**Yokohama-shi Kanagawa-ken (JP)**
Inventor: **Niki, Katsumi**
**No. 243, Mamedo-cho Kohoku-ku**
**Yokohama-shi Kanagawa-ken (JP)**

(74) Representative: **Bond, Bentley George et al**
**Haseltine Lake & Co. 28 Southampton Buildings**
**Chancery Lane**
**London WC2A 1AT (GB)**

(56) References cited:
**ANALYTICAL CHEMISTRY, vol. 55, no. 8, July 1983, pages 1429-1431 N. OYAMA et al.: "Incorporation of redox polymers to polyelectrolyte-coated electrode surfaces"**
**IBM TECHNICAL DISCLOSURE BULLETIN, vol. 22, no. 11, April 1980, pages 5142-5145 E.M. ENGLER et al.: "Electrochemical selectivity of electroactive polymer-coated electrodes"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 109 767 B1

Courier Press, Leamington Spa, England.

## Description

This invention relates to a novel electrode obtained from an electrically conducting solid electrode by modification of the surface thereof, and to applications of the novel electrode.

It is known that electrically conducting solid electrode surfaces can be modified by redox polymers. However, the activity of this modified electrode varies with the degree of polymerization of the redox polymer, and the electrode is active only in strong acidic solutions because electron mediators can be coupled with the polymers only in strong acidic solutions.

An electrode which has polyvinyl pyridine, for example, immobilized on pyrolytic graphite is known (N. Oyama et al., J. Electrochem. Soc., 127, 640 (1980)). It has the disadvantage that effective use thereof is attained only in strongly acidic solutions and that the substances which can be immbolized thereon are limited to those of cationic type.

It is also known from Doklady Akademii Nauk SSSR, *225,* 105—108 (1975) and from "Electrochemical Studies of Biosystems", D. T. Sawyer, A.C.S. Symposium Series, 143 (1977), that electron mediators such as methyl viologen can be used in the form of a solution or a suspension with an unmodified electrode or with a chemically modified electrode. In this case, a large amount of electron mediator has to be used and, in some cases, an electrochemically inactive material, which may decrease the current efficiency, has to be added to the solution.

In DE—A—3148366, there is disclosed an electrode for use in a bioelectrocatalytic process, the electrode consisting of an electrically conductive substance, e.g. pyrolytic graphite, having a chemical binding species, e.g., a bipyridyl type compound, dispersed therein.

In J. Electroanal. Chem. *130,* 199—207 (1981), the immobilization of phenazine methosulphate and phenazine ethosulphate on graphite electrodes is disclosed.

In US—A—4144143, there is disclosed the formation of a stable, apparently polymeric, film of 1,1′-dimethyl-4,4-dipyridyl dichloride on a minigrid electrode.

An object of this invention is to provide a novel modified electrode which overcomes the various disadvantages suffered by the prior art, which is easy to manufacture and handle, and which finds extensive utility in a variety of applications.

The inventors of the present invention have already made earlier inventions, including one which relates to an electrode characterized by having an aromatic group-containing strongly acidic cation exchange resin and a nitrogen-containing electron mediator immobilized on the surface of a solid conductive electrode, and to an electrochemical biosensor using the electrode. This particular invention has emanated from the discovery that a modified electrode having a nitrogen-containing electron mediator stably immbilized thereon can be obtained through the medium of an aromatic group-containing strongly acidic cation exchange resin (see EP—A—0088592 published on 14.09.83).

The inventors, after a further study, have found that a stable chemically modified electrode is easily obtained by selecting, as an electron mediator capable of responding to a biological substance without the intervention of any such aromatic group-containing strongly acidic cation exchange resin, a compound having a particular chemical structure, namely viologen dye, bipyridine, phenanthroline or 1-methoxy-5-methylphenazinium methyl sulphate, and by combining this mediator compound with pyrolytic graphite exposing the basal plane of graphite. The inventors have found that his modified electrode exhibits an excellent electrochemical response to biological substances such as hemoproteins, NADH, HADPH and the like.

Thus, according to the present invention, there is provided a chemically modified electrode comprising an electrically conducting solid electrode of pyrolytic graphite whose basal plane is exposed, and a redox/adsorption electron mediator, characterised in that said redox/adsorption electron mediator is immbolized by adsorption onto a surface of said electrically conducting electrode, and in that said redox/adsorption electron mediator is viologen dye, bipyridine, phenanthroline or 1-methoxy-5- methylphenazinium methyl sulphate.

Heretofore, quinones have often been used as electron mediators for NADH and the like. But prior-art compounds such as that having the formula:

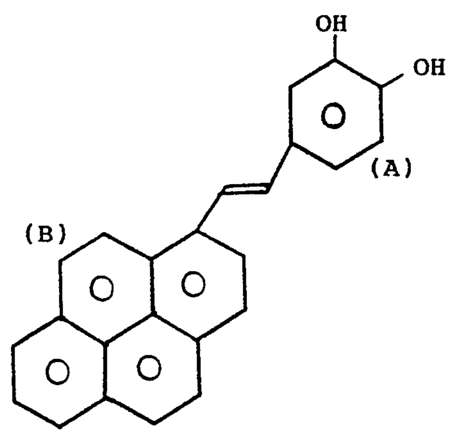

in which the redox site (A) and the adsorption site (B) are spaced apart from one another in the molecule, will not readily oxidize NADH because of their high overvoltage (T. Kuwana et al., J.A.C.S., *105,* 1805 (1983)).

The expression "the adsorption site" is herein used to mean "the adsorption site at which the molecule is adsorbed, i.e. is immobilized by adsorption, on the surface of an electrically conducting solid electrode". The expression "the redox site" is self-explanatory.

On the contrary, the electron mediator compounds used in this invention, which are immobilized on the surface of an electrically conducting solid electrode which is pyrolytic graphite exposing the basal plane of graphite, have a particular chemical structure in which the redox site and the adsorption site are not spaced apart from one another, i.e. both sites are fused, in the molecule. Thus, the mediator compounds used in this invention show both a redox function and a adsorption function, but both functions are not attributable to definitely different sites in the molecule. Thus, the molecules are not only irreversibly adsorbed onto the basal plane of the graphite but also are markedly stabilized. Electron mediators with such a particular chemical structure have the advantage that their over-voltage for NADH and the like is very low. The (electron) mediator (compound) used in this invention having the above-explained particular chemical structure may hereinafter be referred to simply as "a redox/adsorption (electron) mediator (compound)".

For use in the present invention, any of the electrically conducting solid electrodes known in the art may be adopted. A carbon electrode of pyrolytic graphite abundantly exposing the basal plane of graphite such as, for example, the stress-annealed pyrolytic graphite made by U.C.C. (hereinafter referred to as "SAPG) may be cited as a desirable representative.

The modified electrode of this invention can be easily produced by immersing a pyrolytic graphite carbon electrode exposing the basal plane of graphite as described above in an aqueous solution of the redox/adsorption electron mediator and, when necessary, subsequently washing and drying the impregnated carbon electrode. The amount of the redox/adsorption electron mediator to be immobilized on the surface of the carbon electrode is preferably sufficient to form a monomolecular layer (i.e. an amount equivalent to saturated adsorption), although even when this amount is lower than the level required for saturated adsorption, the modified electrode can be effectively used.

The electrode of this invention is better than conventional countertypes in the following respects:

(1) The manufacture of the electrode is extremely simple because it only requires immersion of the carbon electrode in an aqueous solution of a redox/adsorption electron mediator. In addition, the consumption of the electron mediator during this immersion is notably small.

(2) The solution to be used for the electrode reaction is only required to possess a hydrogen ion concentration in the neighbourhood of neutrality, specifically in the pH range of 4 to 10. In other words, according to the present invention, a strongly acidic solution, which is used in making a conventional electrode and which gives rise to problems, is not required. Thus, the modified electrode of the present invention is advantageously used as an electrode capable of responding to biological substances.

(3) The redox/adsorption electron mediator may be effectively used in the form of a monomer and can be quantitatively immobilized. Thus, there is no need to prepare a polymer or to render uniform the degree of polymerization.

(4) Since the redox/adsorption electron mediator is immobilized in a monomolecular layer and retained stably in that state, the substance is consumed in a smaller amount and with greater efficiency than when it is used in the form of a solution or suspension.

(5) The modified electrode is practical and convenient in actual use because it is moderately hydrophilic and not susceptible to dissolution.

As an electrode for fulfilling its function by responding to a biological substance, therefore, the modified electrode of the present invention proves to be an outstanding choice.

Since the electrode of the present invention is capable of generating an electrical current in the presence of a biological substance liable to conjugation with the aforementioned redox/adsorption electron mediator, it can be advantageously utilized as an electrode in a bioreactor. In this case, since the concentration of biological substance and the magnitude of the electrical current generated are in a linear relationship, the modified electrode of the present invention can be utilized as a biosensor capable of the quantitative analysis of biological substances.

To be specific, the concentration of a specific biological substance in a given sample can be determined by fractionally separating the biological substance from the sample, for example by chromatography, by then adding the isolated biological substance to a buffer solution of pH 4 to 10, by placing the modified electrode of this invention in the resultant solution, by placing another appropriate electrode as the counter electrode in the solution, by applying an electrical potential to the opposed electrodes, and by measuring the magnitude of the electrical current consequently generated therein.

Examples of biological substances which respond to the redox/adsorption electron mediator include NADH, NADPH, cytochrome $C_3$, cytochrome C, hemoglobin, chlorophyll, and electron-transfer systems containing these substances.

The present invention will now be illustrated by the following Examples.

### Example 1

An SAPG electrode 5 mm in diameter was immersed for about 10 seconds in a 1 mM aqueous solution of 1-methoxy-5-methylphenazinium methyl sulphate and then washed and dried to produce a modified electrode.

With the use of this electrode, NADH was electrochemically oxidized. During the oxidation, the concentration of NADH and the magnitude of the electrical current generated were measured. The results showed a linear relationship between the two quantities as indicated in Table 1.

Table 1

| Concentration of NADH (g/litre) | Peak current (µA) |
|---|---|
| 0.68 | 12.6 |
| 0.50 | 9.1 |
| 0.35 | 6.3 |
| 0.17 | 3.3 |
| 0.0 | 0.0 |

Example 2

An SAPG electrode 5 mm in diameter was immersed for about 10 seconds in a 1 mM aqueous solution of viologen dye and subsequently washed and dried to produce a modified electrode.

With the use of the electrode, reduced hemoglobin was electrochemically oxidized. During the oxidation, the concentration of hemoglobin and the magnitude of the electrical current generated were measured. The results showed a linear relationship between the two quantities as indicated in Table 2.

Table 2

| Hemoglobin (g/litre) | Peak Current (µA) |
|---|---|
| 12.9 | 3.0 |
| 8.5 | 2.0 |
| 6.5 | 1.5 |
| 3.3 | 0.8 |
| 0.0 | 0.0 |

Example 3

An SAPG electrode 5 mm in diameter was immersed for about 10 seconds in a 1 mM aqueous solution of bipyridine and washed and dried to produce a modified electrode.

With the use of this electrode, ferrous cytochrome C was electrochemically oxidized. During the oxidation, the concentration of cytochrome C and the magnitude of the electrical current generated were measured. The results showed a linear relationship between the two quantities as indicated in Table 3.

Table 3

| Cytochrome C (g/litre) | Peak current (µA) |
|---|---|
| 8.0 | 9.1 |
| 6.0 | 6.9 |
| 4.0 | 4.6 |
| 2.0 | 2.3 |
| 0.0 | 0.0 |

Example 4

When a modified electrode produced by following the procedure of Example 1 was immersed in an aqueous solution containing NADH in a concentration of 50 g/litre and an electrical potential was applied at a current density of about 10 µA/$cm^2$, NAD was substantially quantitatively obtained (about 90% yield).

Example 5

An SAPG electrode 5 mm in diameter was immersed for about 10 seconds in a 1 mM aqueous solution of methyl viologen and washed and dried to produce a modified electrode.

In 20 ml of distilled water, 3 g of polyvinyl alcohol were amply dispersed at room temperature. The resultant mixture was gradually heated under conditions enabling thorough dissolution of the solute without boiling the mixture. The solution was then gradually cooled to normal room temperature. Thereafter, bovine serum albumin was added, when necessary, to the solution in an amount of 1% based on the weight of polyvinyl alcohol, and dissolved therein. With this solution was mixed 0.28 g of anabaena cylindrica suspension (having a chlorophyll content of about 3% by weight). The resultant mixed solution was uniformly spread in a thin layer on the aforementioned modified electrode and immediately dried in a desiccator kept in a dark room at 0 to 5°C. The dry weight was 2 mg.

In an aqueous phosphate buffer solution (pH 7), the electrode prepared as described above and a platinum electrode were set up. The magnitude of electrical current generated between the electrodes was measured in the presence and absence of light (from a xenon lamp). Thus, the electrical current was found to be 3 µA/$cm^2$ in the presence of light and zero in the absence of light. This fact implies the feasibility of photoelectric conversion.

**Claim**

A chemically modified electrode comprising an electrically conducting solid electrode of pyrolytic graphite whose basal plane is exposed, and a redox/adsorption electron mediator, characterised in that said redox/asdorption electron mediator is immobilized by adsorption onto a surface of said electrically conducting electrode, and in that said redox/adsorption mediator is viologen dye, bipyridine, phenanthroline or 1-methoxy-5-methylphenazinium methyl sulphate.

**Patentanspruch**

Chemisch modifizierte Elektrode, die eine elektrisch leintende feste Elektrode aus pyrolytischem Graphit, deren Grundebene offen liegt, und einen Redox/Adsorptions-Elektronenüberträger umfaßt, dadurch gekennzeichnet, daß der Redox/Adsorptions-Elektronenüberträger durch Adsorption auf einer Oberfläche der elektrisch leitenden Elektrode immobilisiert ist, und dadurch, daß der Redox/Adsorptions-Elektronenüberträger ein Viologenfarbstoff. Bipyridin, Phenanthrolin oder 1-Methoxy-5-methylphenaziniummethylsulfat ist.

**Revendication**

Une électrode modifiée chimiquement comprenant une électrode solide électroconductice en

graphite pyrolytique dont le plan de base est exposé et un médiateur électronique à redox/adsorption, caractérisée en ce que ledit médiateur électronique à redox/adsorption est immobilisé par adsorption sur une surface de ladite électrode électroconductrice, et en ce que ledit médiateur électronique à redox/adsorption est un colorant de viologène, la bipyridine, la phénanthroline ou le méthylsulfate de 1-méthoxy-5-méthylphénazinium.